# EUROPEAN PATENT SPECIFICATION

(11) **EP 3 664 786 B1**
(45) Date of publication and mention of the grant of the patent: **05.10.2022**
(21) Application number: 18844011.9
(22) Date of filing: 08.08.2018
(51) Int. Cl.: A61K 31/10, A61K 31/275, A61P 9/04, A61P 17/00, A61P 19/02, A61P 19/08, A61P 29/00

(54) **METHOD FOR TREATING SCHNITZLER'S SYNDROME**
VERFAHREN ZUR BEHANDLUNG DES SCHNITZLER-SYNDROMS
MÉTHODE DE TRAITEMENT DU SYNDROME DE SCHNITZLER

(30) Priority: 11.08.2017 US 201762544617 P
(43) Date of publication of application: 17.06.2020
(73) Proprietor: Olatec Therapeutics LLC, New York, NY 10065 (US)
(72) Inventor: DINARELLO, Charles, A., New York, NY 10065 (US)
(74) Representative: Schüssler, Andrea
(86) International application number: PCT/US2018/045752
(87) International publication number: WO 2019/032672

(56) References cited:
- WO-A1-2014/190015
- WO-A1-2018/129347
- US-A1- 2012 157 524
- MARCHETTI C ET AL: "The human safe NLRP3 inflammasome inhibitor OLT1177 suppresses joint inflammation in murine models of experimental arthritis", ORAL PRESENTATIONS, 14 June 2017 (2017-06-14), pages 89.2-89, XP055777445,
- "Pilot Study of Dapansutrile Capsules in Schnitzler's Syndrome", ClinicalTrials.gov, 23 July 2018 (2018-07-23), XP055572033, Retrieved from the Internet: URL:https://clinicaltrials.gov/ct2/show/NC T03595371

## Description

### FIELD OF THE INVENTION

The present invention relates to using dapansutrile, or its pharmaceutically acceptable salts for treating Schnitzler's syndrome.

### BACKGROUND

Schnitzler's syndrome is a rare disabling disorder; it is an autoinflammatory syndrome characterized by recurrent episodes of inflammation, which occurs in the absence of autoantibodies and antigen specific T cells. A diagnosis of Schnitzler's syndrome is based upon a thorough clinical evaluation, a detailed patient history, exclusion of other disorders, and identification of characteristic findings, specifically a urticarial rash, an IgM or IgG component and at least two of the following findings: intermittent unexplained fever, arthralgia or arthritis, bone pain, lymphadenopathy, hepato- or splenomegaly, an acute phase response, and abnormal findings on bone morphological investigations (Lipsker et al, Medicine (Baltimore) 2001; 80(1):37-44; Schnitzler et al., Ann Dermatol Venereol 1989; 116(8):547-550). Symptomatic episodes are associated with increased serum concentrations of both pro-inflammatory mediators (TNFα, IL-6, IE1β and IFN-γ) as well as of the antiinflammatory compounds (IL-1ra, sTNFR p55 and sTNFR p75). In vitro and ex vivo experiments suggest a central role in the pathogenesis for IL-1β. In most cases, the syndrome follows a chronic, benign course, but at least 15% will develop a lymphoproliferative disorder in the long term. Treatment remains a challenge and to date, no spontaneous complete remissions have been reported.

The recombinant IL-1Ra anakinra was evaluated as a potential treatment and found to rapidly control all the symptoms of this syndrome. Clinical benefit of anakinra treatment is observed often within a few hours on urticaria and within a few days on C-reactive protein (CRP) levels and leukocytosis. As there are no approved treatments for Schnitzler's syndrome, anakinra has been used off-label. The short half-life of anakinra (4 to 6 hours) requires daily subcutaneous injections, which occasionally lead to strong local injection site reactions.

Canakinumab is a fully human selective monoclonal anti-IL-1β antibody with a half-life of 28 days. It has also been administered off-label monthly to subjects as an effective and well-tolerated therapy for Schnitzler's syndrome (Vanderschueren 2012). Rilonacept (IL-1 inhibitor) has also been shown to reduce clinical symptoms and inflammatory markers in subjects with Schnitzler's syndrome for up to 1 year (Krause 2012). However, given the burden of painful daily (anakinra), weekly (rilonacept), or monthly (canakinumab) injections, high treatment costs for biologies and ineffective alternative treatments, a safe and effective approved oral treatment for Schnitzler's would be an ideal solution for subjects dealing with this rare syndrome.

There is a need to develop methods and compositions for treating Schnitzler's syndrome.

### BRIEF DESCRIPTION OF THE DRAWINGS

FIG. 1 shows the inhibition of IL-1β from human macrophages treated in vitro with dapansutrile.
FIG. 2 shows the levels of IL-1β in synovial membrance of mice treated with dapansutrile or untreated mice.
FIG. 3A shows the results of Western blots for caspase-1 (p45 and p10) of cell lysates (Lys) and supernatant (Sup) from J774A.1 cells following LPS/ATP stimulation in mouse J774A.1 cells. FIG. 3B shows the results of Mean ±SEM of caspase-1 activity in J774A.1 cells lysates following LPS and nigericin (NIG) stimulation in presence of 50µM dapansutrile (OLT1177).

### DETAILED DESCRIPTION OF THE INVENTION

The inventor has discovered that dapansutrile inhibits the oligomerization of the NLRP3 inflammasome, which in turn prevents the activation of caspase-1 and the maturation of pro-IL-1β and pro-IL-18 to their active forms IL-1β and IL-18, respectively. In vitro, ex vivo and in vivo studies have demonstrated that dapansutrile inhibits the processing and release of IL-1β, but not the synthesis of the IL-1β precursor. The inventor has discovered that dapansutrile is effective in improving the symptoms of Schnitzler's syndrome.

The invention is defined in the claims and relates to dapansutrile, or a pharmaceutically acceptable solvate thereof, as a compound for use in a method for treating Schnitzler's syndrome in a subject.

Described is a method for treating Schnitzler's syndrome. The method comprises the step of administering to a subject suffering from Schnitzler's syndrome an effective amount of dapansutrile, or a pharmaceutically acceptable salt thereof, to treat Schnitzler's syndrome. Schnitzler's syndrome and Schnitzler syndrome are used interchangeably in this application.

### Compound

The present invention uses a purified compound of dapansutrile (3-methanesulfonylpropionitrile), or a pharmaceutically acceptable solvate thereof:

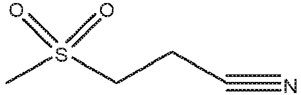

"Pharmaceutically acceptable solvates," as used herein, are sovates that retain the desired biological activity of the parent compound and do not impart undesired toxicological effects. Solvates are addition complexes in which the compound is combined with an acceptable cosolvent in some fixed proportion. Co-solvents include, but are not limited to, water, ethyl acetate, lauryl lactate, myristyl lactate, cetyl lactate, isopropyl myristate, methanol, ethanol, 1-propanol, isopropanol, 1-butanol, isobutanol, tert-butanol, acetone, methyl ethyl ketone, acetonitrile, benzene, toulene, xylene(s), ethylene glycol, dichloromethane, 1,2-dichloroethane, N-methylformanlide N,N-dimethylformamide, N-methylacetamide, pyridine, dioxane, and diethyl ether.

### Pharmaceutical Compositions

Described are pharmaceutical compositions comprising one or more pharmaceutically acceptable carriers and an active compound of dapansutrile, or a pharmaceutically acceptable salt, or a solvate thereof. The active compound or its pharmaceutically acceptable salt or solvate in the pharmaceutical compositions in general is in an amount of about 0.01-20%, or 0.05-20%, or 0.1-20%, or 0.2-15%, or 0.5-10%, or 1-5% (w/w), for a topical formulation; about 0.1-5% for an injectable formulation, 0.1-5% for a patch formulation, about 1-90% for a tablet formulation, and 1-100% for a capsule formulation. The active compound used in the pharmaceutical composition in general is at least 90%, preferably 95%, or 98%, or 99% (w/w) pure.

In one instance, the pharmaceutical composition is in a dosage form such as tablets, capsules, granules, fine granules, powders, syrups, suppositories, injectable solutions, patches, or the like. In another instance, the active compound is incorporated into any acceptable carrier, including creams, gels, lotions or other types of suspensions that can stabilize the active compound and deliver it to the affected area by topical applications. The above pharmaceutical composition can be prepared by conventional methods.

Pharmaceutically acceptable carriers, which are inactive ingredients, can be selected by those skilled in the art using conventional criteria. Pharmaceutically acceptable carriers include, but are not limited to, non-aqueous based solutions, suspensions, emulsions, microemulsions, micellar solutions, gels, and ointments. The pharmaceutically acceptable carriers may also contain ingredients that include, but are not limited to, saline and aqueous electrolyte solutions; ionic and nonionic osmotic agents such as sodium chloride, potassium chloride, glycerol, and dextrose; pH adjusters and buffers such as salts of hydroxide, phosphate, citrate, acetate, borate; and trolamine; antioxidants such as salts, acids and/or bases of bisulfite, sulfite, metabisulfite, thiosulfite, ascorbic acid, acetyl cysteine, cysteine, glutathione, butylated hydroxyanisole, butylated hydroxytoluene, tocopherols, and ascorbyl palmitate; surfactants such as lecithin, phospholipids, including but not limited to phosphatidylcholine, phosphatidylethanolamine and phosphatidyl inositiol; poloxamers and poloxamines, polysorbates such as polysorbate 80, polysorbate 60, and polysorbate 20, polyethers such as polyethylene glycols and polypropylene glycols; polyvinyls such as polyvinyl alcohol and povidone; cellulose derivatives such as methylcellulose, hydroxypropyl cellulose, hydroxyethyl cellulose, carboxymethyl cellulose and hydroxypropyl methylcellulose and their salts; petroleum derivatives such as mineral oil and white petrolatum; fats such as lanolin, peanut oil, palm oil, soybean oil; mono-, di-, and triglycerides; polymers of acrylic acid such as carboxypolymethylene gel, and hydrophobically modified cross-linked acrylate copolymer; polysaccharides such as dextrans and glycosaminoglycans such as sodium hyaluronate. Such pharmaceutically acceptable carriers may be preserved against bacterial contamination using well-known preservatives, these include, but are not limited to, benzalkonium chloride, ethylenediaminetetraacetic acid and its salts, benzethonium chloride, chlorhexidine, chlorobutanol, methylparaben, thimerosal, and phenyl ethyl alcohol, or may be formulated as a non-preserved formulation for either single or multiple use.

For example, a tablet formulation or a capsule formulation of the active compound may contain other excipients that have no bioactivity and no reaction with the active compound. Excipients of a tablet may include fillers, binders, lubricants and glidants, disintegrators, wetting agents, and release rate modifiers. Binders promote the adhesion of particles of the formulation and are important for a tablet formulation. Examples of binders include, but not limited to, carboxymethylcellulose, cellulose, ethylcellulose, hydroxypropylmethylcellulose, methylcellulose, karaya gum, starch, starch, and tragacanth gum, poly(acrylic acid), and polyvinylpyrrolidone.

For example, a patch formulation of the active compound may comprise some inactive ingredients such as 1,3-butylene glycol, dihydroxyaluminum aminoacetate, disodium edetate, D-sorbitol, gelatin, kaolin, methylparaben, polysorbate 80, povidone (polyvinylpyrrolidone), propylene glycol, propylparaben, sodium carboxymethyl cellulose, sodium polyacrylate, tartaric acid, titanium dioxide, and purified water. A patch formulation may also contain skin permeability enhancer such as lactate esters (e.g., lauryl lactate) or diethylene glycol monoethyl ether.

Topical formulations including the active compound can be in a form of gel, cream, lotion, liquid, emulsion, ointment, spray, solution, and suspension. The inactive ingredients in the topical formulations for example include, but not limited to, lauryl lactate (emollient/permeation enhancer), diethylene glycol monoethyl ether (emollient/permeation enhancer), DMSO (solubility enhancer), silicone elastomer (rheology/texture modifier), caprylic/capric triglyceride, (emollient), octisalate, (emollient/UV filter), silicone fluid (emollient/diluent), squalene (emollient), sunflower oil (emollient), and silicone dioxide (thickening agent).

### Method of Use

The present invention is directed to dapansutrile for use in a method of treating Schnitzler's syndrome. The method comprises the steps of first identifying a subject suffering from Schnitzler's syndrome, and administering to the subject dapansutrile, in an amount effective to treat Schnitzler's syndrome. "An effective amount," as used herein, is the amount effective to treat a disease by ameliorating the pathological condition or reducing the symptoms of the disease.

In one embodiment, the method reduces or alleviates the disease symptoms by reducing the frequency, intensity and/or duration of chronic urticarial rash and/or fever in a patient with Schnitzler's syndrome.

In one embodiment, the method reduces the elevated blood CRP level in a patient.

In one embodiment, the method reduces the clinical scores of the disease symptoms. The symptoms on the symptom score list include abdominal pain, nausea, diarrhea, throwing up, joint pain, muscle pain, wounds in the mouth, skin abnormalities, fatigue, headache, throat pain, nose coldness, chest pain, and otherwise symptom. The scores of one day are added up as clinical scores.

A clinical complete remission in a patient is defined as the disappearance of symptomatology (skin rash, malaise) with a clinical score <5 in the absence of fever (temperature > 38.0°C), and normalization of CRP (< 10 mg/L) and WBC (< 11×10⁹/L). The skin rash is documented photographically.

In one embodiment, the method improves one or more of the following symptoms: intermittent unexplained fever, arthralgia or arthritis, bone pain, lymphadenopathy, hepato- or splenomegaly, an acute phase response, abnormal findings on bone morphological investigations, and abnormal CRP (C-reactive protein) level.

The pharmaceutical composition used in the present invention can be applied by systemic administration and local administration. Systemic administration includes oral, parenteral (such as intravenous, intramuscular, subcutaneous or rectal), and other systemic routes of administration. In systemic administration, the active compound first reaches plasma and then distributes into target tissues. Local administration includes topical administration.

Dosing of the composition can vary based on the extent of the injury and each patient's individual response. For systemic administration, plasma concentrations of the active compound delivered can vary; but are generally 0.1-1000 µg/mL or 1-100 µg/mL.

In one instance, the pharmaceutical composition is administrated orally to the subject. The dosage for oral administration is generally at least 0.5 mg/kg/day and less than 100 mg/kg/day. For example, the dosage for oral administration is 0.5-100, or 1-50, or 2-50 mg/kg/day, for a human subject. For example, the dosage for oral administration is 50-10000 mg/day, and preferably 100-5000, 100-3000, 500-2000, 500-4000 mg/day for a human subject. The drug can be orally taken once, twice, three times, or four times a day.

In one instance, the pharmaceutical composition is administrated intravenously to the subject. The dosage for intravenous bolus injection or intravenous infusion is generally 0.03 to 20 or 0.03 to 10 mg/kg/day.

In one instance, the pharmaceutical composition is administrated subcutaneously to the subject. The dosage for subcutaneous administration is generally 0.3-20 or 0.3-3 mg/kg/day.

Those of skill in the art will recognize that a wide variety of delivery mechanisms are also suitable for the present invention.

The present invention may be used in combination with one or more other treatments, e.g., thalidomide, steroids, anakinra, and canakinumab. Dapansutrile can be co-administered with thalidomide, steroids, anakinra, and canakinumab to a patient suffering from Schnitzler's syndrome, simultaneously or sequentially.

The present invention is useful in treating a mammal subject, such as humans, horses, and dogs. The present invention is particularly useful in treating humans.

The following examples further illustrate the present invention. These examples are intended merely to be illustrative of the present invention and are not to be construed as being limiting.

### EXAMPLES

### Example 1. Suppression of IL-1β release from human monocytes-derived macrophages

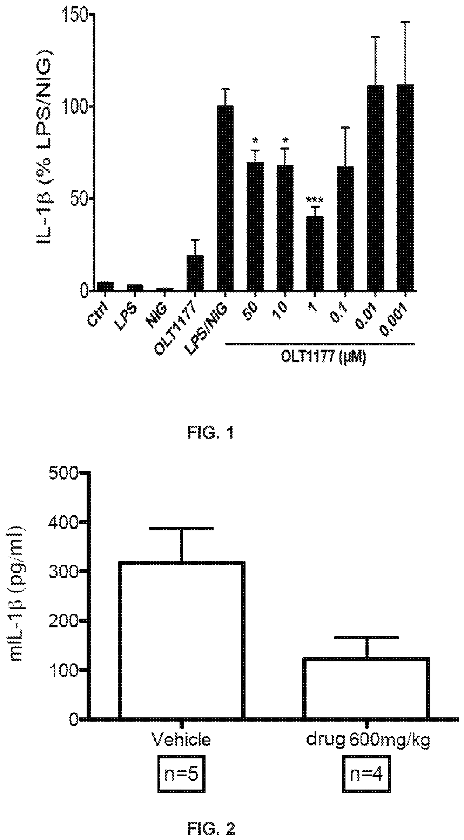

Peripheral blood mononuclear cells (PBMCs) were isolated from blood of 11 healthy human donors and differenciates in macrophages using GM-CSF (5ng/ml) for 5 days. They were stimulated with 1µg/mL with lipopolysaccharide (LPS, priming phase) at time zero. At 30 minutes, they were incubated with different concentrations of dapansutrile (OLT1177) from 0.001-50 µM. At 4 hours, 10 µM of nigericin (NIG, release phase) was added. At the end of an 5 hour incubation period, the supernatant was harvested and assayed for IL-1β using respective ELISA kits following manafacturer's protocols. The results are shown in FIG. 1.

FIG. 1 shows data on the suppression of IL-1β release from human monocytes-derived macrophages, following stimulation with lipopolysaccharide (priming phase) and nigericin (release phase). Maximum suppression effect was observed at 1 µM of dapansutrile.

The results show that dapansutrile suppresses the release of IL-1β from stimulated monocytes-derived macrophages. ^{∗∗∗}: P<0.001, ^{∗}: P<0.05, compared to control.

### Example 2. Mouse IL-1β Level

Zymosan injected directly into the knee joint of mice elicits an inflammatory response and is used as a model of arthritis (Verschure et al, Ann. Rheum Dis. 53:455-460, 1994). Endpoints measured in this model include knee joint swelling score, cytokine levels in the synovial tissue and microscopic pathology of the knee.

Arthritis was induced in mice by intra-articular injection of zymosan (180µg) into the knee joint (at time zero) of both knees in each mouse. Mice were treated by oral gavage with vehicle (water, n=5) or dapansutrile in vehicle (600 mg/kg, n=4) at times -25hr, -13hr, and -1hr. After zymosan injection at time zero, mice were further treated with vehicle or dapansutrile (600 mg/kg) at times +11hr and +23hr. Mice were sacrificed 24 hours following the Zymosan intraarticulr idministration.

Macroscopic joint swelling was assessed on all knees after the skin is removed using a scoring system ranging from 0 to 3, with 0 being no swelling and 3 being severe swelling. The results showed that dapansutrile-treated mice had a significant reduction in the joint swelling, when comparing with vehicle-treated mice.

In addition, synovial membrane was taken from one knee per mouse and the cells were lysed for measurement of mouse IL-1β, using an ELISA kit from R&D Systems, Inc., following manufacturer's protocols. The results are shown as mean ± standard error of mean and statistical evaluation is performed. FIG. 2 shows that the level of IL-1β in synovial membrane was significantly reduced in mice treated with dapansutrile comparing with untreated mice.

The remaining knees were also processed for microscopic pathology for assessment of cellular influx into the site of inflammation.

### Example 3. Inhibition of Caspase-1 Activation by Dapansutrile

J774A.1 murine macrophages cells were stimulated with 1 µg/ml lipopolysaccharide (LPS) for 4 hours and either nigericin (NIG, 10 µM) or ATP (5 mM) was added for 1 hour. OLT1177 was added to the cells either 30 minutes following LPS stimulation or at the same time as ATP.

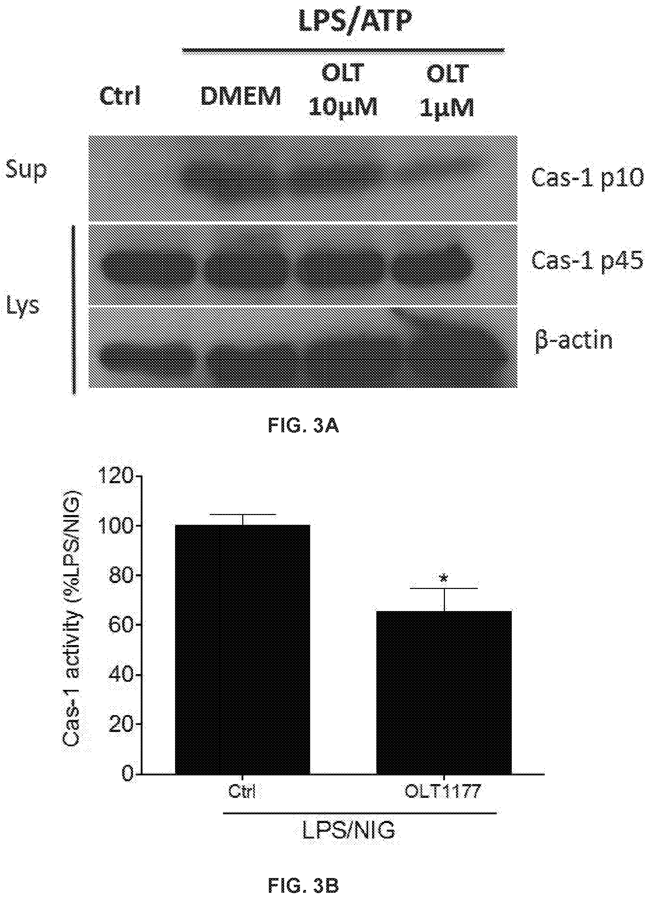

FIG. 3A shows the results of Western blots for caspase-1 (p45 and p10) of cell lysates (Lys) and supernatant (Sup) from J774A.1 cells following LPS/ATP stimulation in mouse J774A.1 cells. A subunit of activated caspase-1 (p10) was reduced in J774A.1 cells in the presence of dapansutrile (OLT)

FIG. 3B shows the results of Mean ±SEM of caspase-1 activity in J774A.1 cells lysates following LPS and NIG stimulation in presence of 50µM dapansutrile (OLT1177). Caspase-1 activity is expressed as percent change of the mean value of each assay (n=3) with LPS/NIG set at 100%. Caspase-1 (Cas-1) activity was reduced by 35% (p<0.05) in an enzymatic assay of cell lysate from stimulated cells in the presence of dapansutrile, comparing with control.

The results show that dapansutrile (OLT1177) inhibited caspase-1 activation in J774A.1 cell line.

### Example 4. Clinical Study

### Objectives:

1. To assess the safety, tolerability, and pharmacokinetics of dapansutrile (capsules or tablets) after oral administration in subjects with Schnitzler's syndrome
2. To assess the clinical activity of dapansutrile, including the change in symptoms of Schnitzler's syndrome, upon withdrawal of anakinra therapy
3. To assess the pharmacodynamics and changes in inflammatory biomarkers after oral
   administration of dapansutrile capsules, upon withdrawal of anakinra therapy, and after all therapy has ended

### Methodology:

This is an open-label, single-center, repeat dose, single cohort, proof-of-concept, safety, pharmacodynamics and efficacy study of dapansutrile in subjects with Schnitzler's syndrome (SchS). Five to ten subjects are enrolled.

The study is designed to determine that the administration of dapansutrile prevents a relapse of the symptoms of Schnitzler's syndrome after cessation of anakinra therapy. For the first 14 study days, patient's standard dose of anakinra is co-administered. and is gradually reduced until complete removal of anakinra therapy by study day 15.

### Main Criteria for Inclusion:

1) Male and female subjects 18 years old or older
2) Prior diagnosis of Schnitzler's syndrome; characterized by a chronic urticarial rash and monoclonal gammopathy, accompanied by at least two of the following features: intermittent unexplained fever, arthralgia or arthritis, bone pain, lymphadenopathy, hepato- or splenomegaly, an acute phase response, abnormal findings on bone morphological investigations and an increase in WBC count.
3) Presence of Schnitzler's syndrome that is well controlled by and responsive to anakinra for at least 6 weeks prior to the Screening/Baseline visit
4) Grade 0 SchS symptoms (see Table 1) at the Screening/Baseline visit

**Table 1 SchS Symptom Index Grading Scale**

| **Grade** | **Description** | **Criteria** |
|---|---|---|
| 0 | No symptoms of Schnitzler's syndrome | 1. An Investigator Global Assessment of Disease Activity score of 0 or 1; and |
| | | 2. Normal CRP level (≤ 10 mg/L) |
| 1 | Mild symptoms of Schnitzler's syndrome | 1. An Investigator Global Assessment of Disease Activity score of 0 or 1; and |
| | | 2. CRP > 10 mg/L but ≤ 30 mg/L |
| 2 | Moderate symptoms of Schnitzler's syndrome | 1. An Investigator Global Assessment of Disease Activity score of 1 or 2; and |
| | | 2. CRP > 30 mg/L but ≤ 50 mg/L (without explanation for elevated CRP other than Schnitzler's syndrome) |
| 3 | Severe symptoms of Schnitzler's syndrome | 1. An Investigator Global Assessment of Disease Activity score of 2 or higher; and/or |
| | | 2. CRP of > 50 mg/L (without explanation for elevated CRP other than Schnitzler's syndrome) |

5) Acceptable overall medical condition to be safely enrolled in and to complete the study in the opinion of the Investigator

### Main Criteria for Exclusion:

1) Pregnant, nursing or intent to become pregnant during the study
2) Not responsive or well controlled by anakinra therapy for at least 6 weeks prior to the Screening/Baseline visit
3) Use or planned use of any prohibited concomitant medications/therapies such as immunotherapies or corticosteroids during the study (until relapse and resumption of anakinra injections)
4) Active infection within 3 days prior to the Screening/Baseline visit
5) History of or known positive for HIV, Hepatitis B surface antigen (HBsAg) or antibodies to Hepatitis C Virus (HCV)
6) Any other concomitant medical or psychiatric conditions, including alcohol or substance abuse, diseases or prior surgeries that in the opinion of the Investigator would impair the subj ect from safely participating in the trial and/or completing protocol requirements

### Dose and Mode of Administration:

Dapansutrile capsules are self-administered by mouth twice each day (BID) beginning at the Screening/Baseline (Day 1) visit and continuing for a period of 30 days. For up to the first 14 days of dapansutrile therapy, the patient's standard daily dose of anakinra (50-200 mg) is co-administered subcutaneously with dapansutrile. The dosing of anakinra is gradually reduced until complete removal of anakinra therapy by study day 15.

Subjects takes dapansutrile by mouth twice each day (BID) approximately 12 hours apart for a total of 500 mg - 5 g of dapansutrile per day for the first week, then 200 mg - 2 g of dapansutrile per day for the remaining treatment period. Exact timing for reduction of dapansutrile is determined following the Investigator's assessment after each clinic visit by the patient.

At the end of the treatment period, subjects remain off all medication for Schnitzler's syndrome unless a relapse of symptoms occurs, at which time subjects visit the study clinic (the "Symptom Onset visit") and consult with the Investigator to determine when to restart anakinra therapy.

### Clinical Trial Duration:

The trial duration is up to 60 days for all subjects enrolled, which consists of up to 11 visits: Screening/Baseline (Day 1), Day 8, Day 15, Day 22, Day 29, Day 30, Day 31, Day 32, Day 36, Day 50 and Day 57.

### Safety Criteria for Evaluation:

Safety criteria for evaluation are as follows:
- Physical examination
- Vital Signs (pulse, resting blood pressure, temperature, respiration rate)
- Safety laboratory measures (chemistry, hematology and urinalysis)
- Adverse Events during the clinical trial

### Clinical Activity Outcomes for Evaluation:

Primary efficacy outcome is:
   - The proportion of subjects with Grade 0 or 1 SchS symptoms at the Day 30 visit
Secondary efficacy outcome measures are:
   - Photographs of the posterior torso and/or other non-identifying areas of the body that display(ed) urticarial rash at Baseline
   - Investigator global assessment of disease activity (5-pointNRS)
   - Subject-completed study diaries
      - Subject Global Assessment of Disease Activity (11-point NRS)
      - Subject Skin Assessment (5-point NRS)
      - Ear (tympanic) body temperature measurements
   - Time to relapse of SchS symptoms after cessation of dapansutrile capsules as defined by the emergence of Grade 2 or higher SchS symptoms
   - Subject global evaluation of treatment
Pharmacokinetic/Pharmacodynamics outcome measures are:
   - Pharmacodynamics biomarkers:
      - C-reactive protein (CRP)
      - Plasma cytokines, including: IL-1β, IL-1Ra, IL-18, IL-6, TNF-α, sTNFR p55, and sTNFR p75
      - Urinary neopterin (normalized for urinary creatinine)
      - Serum Amyloid A (SAA)
      - S100 proteins: S100A8, S100A9 and S100A12
      - Ex vivo analysis of blood samples
   - Pharmacokinetics
      - Plasma concentrations of dapansutrile

The invention, and the manner and process of making and using it, are now described in such full, clear, concise and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point out and distinctly claim the subject matter regarded as invention, the following claims conclude the specification.

## Claims

1. Dapansutrile, or a pharmaceutically acceptable solvate thereof, as a compound for use in a method for treating Schnitzler's syndrome in a subject.

2. The compound for the use according to Claim 1, wherein said method reduces the frequency, intensity and/or duration of chronic urticarial rash and fever in the subject.

3. The compound for the use according to Claim 1, wherein said method improves one or more of the following symptoms: intermittent unexplained fever, arthralgia or arthritis, bone pain, lymphadenopathy, hepato- or splenomegaly, an acute phase response, abnormal findings on bone morphological investigations, and elevated CRP level.

4. The compound for the use according to Claim 1, wherein said compound is administered by systemic administration.

5. The compound for the use according to Claim 4, wherein said compound is administered by oral administration.

## Patentansprüche

1. Dapansutril oder ein pharmazeutisch annehmbares Solvat davon als eine Verbindung zur Verwendung in einem Verfahren zur Behandlung des Schnitzler-Syndroms bei einem Patienten.

2. Verbindung zur Verwendung nach Anspruch 1, wobei das Verfahren die Häufigkeit, Intensität und/oder Dauer des chronischen urtikariellen Ausschlags und des Fiebers bei dem Patienten verringert.

3. Verbindung zur Verwendung nach Anspruch 1, wobei das Verfahren eines oder mehrere der folgenden Symptome verbessert: intermittierendes unerklärliches Fieber, Arthralgie oder Arthritis, Knochenschmerzen, Lymphadenopathie, Hepato- oder Splenomegalie, eine Akute-Phase-Reaktion, abnorme Befunde bei knochenmorphologischen Untersuchungen und ein erhöhter CRP-Spiegel.

4. Verbindung zur Verwendung nach Anspruch 1, wobei die Verbindung durch systemische Verabreichung verabreicht wird.

5. Verbindung für die Verwendung nach Anspruch 4, wobei die Verbindung durch orale Verabreichung verabreicht wird.

## Revendications

1. Dapansutrile, ou un solvate pharmaceutiquement acceptable de celui-ci, en tant que composé destiné à être utilisé dans une méthode de traitement du syndrome de Schnitzler chez un sujet.

2. Composé pour l'utilisation selon la revendication 1, dans lequel ladite méthode réduit la fréquence, l'intensité et/ou la durée de l'éruption urticarienne chronique et de la fièvre chez le sujet.

3. Composé pour l'utilisation selon la revendication 1, dans lequel ladite méthode améliore un ou plusieurs des symptômes suivants : fièvre intermittente inexpliquée, arthralgie ou arthrite, douleur osseuse, lymphadénopathie, hépato- ou splénomégalie, une réponse de phase aiguë, des résultats anormaux sur les investigations morphologiques osseuses, et un niveau élevé de CRP.

4. Composé pour l'utilisation selon la revendication 1, dans lequel ledit composé est administré par voie systémique.

5. Composé pour l'utilisation selon la revendication 4, dans lequel ledit composé est administré par voie orale.
